# EUROPEAN PATENT SPECIFICATION

(11) **EP 2 412 796 B1**
(45) Date of publication and mention of the grant of the patent: **06.08.2014**
(21) Application number: 10755519.5
(22) Date of filing: 18.03.2010
(51) Int. Cl.: C12N 1/12, C07K 14/405

(54) **METHOD FOR THE OBTENTION AND MASS PRODUCTION OF AN ISOLATED CYANOBACTERIA SPECIES PRODUCING PARALYSING PHYCOTOXINS**
VERFAHREN ZUR GEWINNUNG UND MASSENPRODUKTION EINER ISOLIERTEN CYANOBAKTERIENSPEZIES ZUR HERSTELLUNG LÄHMENDER PHYCOTOXINE
PROCÉDÉ D'OBTENTION ET DE PRODUCTION DE MASSE D'UNE ESPÈCE DE CYANOBACTÉRIE ISOLÉE PRODUCTRICE DE PHYCOTOXINES PARALYSANTES

(30) Priority: 24.03.2009 CL 7222009
(43) Date of publication of application: 01.02.2012
(73) Proprietor: Proteus S.A., Santiago (CL)
(72) Inventor: LAGOS GONZÁLEZ, Marcelo Santiago, Providencia Santagio 7530091 (CL)
(74) Representative: Carvajal y Urquijo, Isabel
(86) International application number: PCT/IB2010/051188
(87) International publication number: WO 2010/109387

(56) References cited:
- POMATI F ET AL: "Evidence for differences in the metabolism of saxitoxin and C1+2 toxins in the freshwater cyanobacterium Cylindrospermopsis raciborskii T3", BIOCHIMICA ET BIOPHYSICA ACTA - GENERAL SUBJECTS, ELSEVIER SCIENCE PUBLISHERS, NL, vol. 1674, no. 1, 6 September 2004 (2004-09-06), pages 60-67, XP004549459, ISSN: 0304-4165, DOI: 10.1016/J.BBAGEN.2004.05.006
- POMATI ET AL: "The purine degradation pathway: Possible role in paralytic shellfish toxin metabolism in the cyanobacterium Planktothrix sp", ENVIRONMENT INTERNATIONAL, PERGAMON PRESS, US, vol. 27, no. 6, 1 December 2001 (2001-12-01), pages 463-470, XP008141008, ISSN: 0160-4120, DOI: 10.1016/S0160-4120(01)00101-5 [retrieved on 2001-11-26]
- SHIMIZU YUZURU: "Toxigenesis and biosynthesis of saxotoxin analogues", PURE AND APPLIED CHEMISTRY, vol. 58, no. 2, 1986, pages 257-262, XP002690593, UK
- SOTO ET AL: "The effects of chloramphenicol, arginine and temperature on PST-production by Cylindrospermopsis raciborskii strain D9", PROCEEDINGS OF THE 12TH INTERNATIONAL CONFERENCE ON HARMFUL ALGAE. COPENHAGEN 2008, INTERNATIONAL SOCIETY FOR THE STUDY OF HARMFUL ALGAE AND INTERGOVERNMENTAL OCEANOGRAPHIC COMMISSION OF UNESCO, 1 January 2008 (2008-01-01), pages 330-333, XP008141004,
- SHIMIZU Y ET AL: "BIOSYNTHESIS OF SAXITOXIN ANALOGS THE UNEXPECTED PATHWAY", JOURNAL OF THE AMERICAN CHEMICAL SOCIETY, vol. 106, no. 21, 1984, pages 6433-6434, XP002690594, ISSN: 0002-7863
- QUINTERO ET AL: "Arginine catabolism in the cyanobacterium Synechocystis sp. Strain PCC 6803 involves the urea cycle and arginase pathway.", JOURNAL OF BACTERIOLOGY, vol. 182, no. 4, 1 February 2000 (2000-02-01), pages 1008-1015, XP055050113, ISSN: 0021-9193
- 'Proceedings of the 12th International Conference on Harmful Algae. International Society for the Study of Harmful Algae and Intergovernmental Oceanographic Commission of UNESCO, Copenhagen 2008.', article SOTO, K. ET AL.: 'The effects of chloramphenicol, arginine and temperature on PST-production by Cylindrospermopsis raciborskii strain D9.', pages 330 - 333, XP008141004
- POMATI, F. ET AL.: 'Evidence for differences in the metabolism of saxitoxin and C1+2 toxins in the freshwater cyanobacterium Cylindrospermopsis raciborskii T3.' BIOCHIMICA ET BIOPHYSICA ACTA vol. 1674, no. 1, September 2004, pages 60 - 67, XP004549459
- POMATI, F. ET AL.: 'The purine degradation pathway: Possible role in paralytic shellfish toxin metabolism in the cyanobacterium Planktothrix sp.' FPL. ENVIRONMENT INTERNATIONAL. vol. 27, no. 6, 2001, pages 463 - 470, XP008141008
- LONG, B. M. ET AL.: 'Cellular microcystin content in N-limited Microcystis aeruginosa can be predicted from growth rate.' APPLIED AND ENVIRONMENTAL MICROBIOLOGY. vol. 67, no. 1, January 2001, pages 278 - 283, XP008140347
- 'Media recipes used in CMAR.' CMAR METHODS., [Online] 02 August 2008, XP008141010 Retrieved from the Internet: <URL:http://www.marine.csiro.au/ microalgae/methods/Media%20CMARC%20recipes. htm> [retrieved on 2010-05-19]

## Description

### FIELD OF THE INVENTION

This invention is related to a method to produce cyanobacteria (photosynthetic blue-green algae), specifically to the development of a massive artificial process operating under controlled conditions that is useful to generate desired metabolites, such as paralyzing toxins, at industrial levels. The method is characterized by its high efficiency, high yield, simplicity and high profitability.

### DESCRIPTION OF THE PREVIOUS ART

Neosaxitoxin and saxitoxin are components of the paralyzing poison produced by dinoflagellate species of the genera *Alexandrium* sp., *Piridinium* sp. and *Gimnodinium* sp., and therefore they are sometimes present in filter-feeding bivalves and carnivore gastropods contaminated by harmful algal blooms, commonly known as red tides (Lagos, N. (1998) Microalgal blooms: a global issue with negative impact in Chile. Biol. Res. 31: 375-386). These phycotoxins are produced not only by marine dinoflagellates, but also by fresh-water cyanobacteria (photosynthetic blue-green algae).

There are only 5 cyanobacteria described in the literature able to produce shellfish paralyzing phycotoxins, each of them having a characteristic composition regarding the amount and type of produced phycotoxins (profile of paralyzing phycotoxins):
- ***Cylindrospermopsis raciborskii**,* isolated from Brazil (Lagos, N., Onodera, H., Zagatto, P.A., Andrinolo, D., Azevedo, S.M.F.Q., and Oshima, Y., 1999, The first evidence of paralytic shellfish toxins in the freshwater cyanobacterium Cylindrospermopsis raciborskii, isolated from Brazil. TOXICON, 37: 1359 - 1373; Molica, R., Onodera, H., Garcia, C., Rivas, M., Andrinolo, D., Nascimento, S., Meguro, H., Oshima, Y., Azevedo, S., and Lagos, N., 2002, Toxins in the freshwater cyanobacterium Cylindrospermopsis raciborskii, isolated from Tabocas reservoir in Caruaru, Pernambuco, Brazil, Phycologia, 41 (6): 606 - 611).
- ***Aphanizomenon flos-aquae**,* isolated from Portugal (Pereira, P., Onodera, H., Andrinolo, D., Franca, S., Araujo, F., Lagos, N., and Oshima, Y., 2000, Paralytic shellfish toxins in the freshwater cyanobacteria Aphanizomenon flos-aquae, isolated from Montargil reservoir, Portugal. Toxicon, 38: 1689 - 1702).
- ***Anabaena circinalis**,* isolated from Australia (Lagos, 1999).
- ***Lyngbya wollei,*** isolated from North America (Lagos, 1999).
- ***Aphanizomenon(Aph) gracile* (Lemm) Lemm** (Pereira P LMECYA40). This photosynthetic unicellular blue-green alga is the only cyanobacteria of this type that only produces tetrahydropurines: neosaxitoxin and saxitoxin, in a proportion of 78 to 82% neosaxitoxin and 22 to 18% saxitoxin.

The method proposed in the present application is a continuous culture operating under controlled conditions of a cyanobacterium that produces a simple profile of paralyzing phycotoxins. This profile depends on the cultured species, and includes neosaxitoxin and saxitoxin or gonyaulatoxins 2 and 3. Furthermore, this culture method includes micronutrient addition that allows higher production rates of these pharmacologically active compounds per wet weight of the algae than those previously described.

Neosaxitoxin, saxitoxin and gonyaulatoxins are compounds that have a tetrahydropurine basic structure and are produced by the abovementioned cyanobacteria as secondary metabolites. These compounds, as all secondary metabolites, are inside the cells but are also released into the culture medium. In this way, two sources of these products are generated: in the cell pellet fraction and in the medium where cyanobacteria are cultured.

The massive continuous culture of these cyanobacteria, both in open or naturally-illuminated reactors and in closed illuminated reactors where light is provided by artificial means such as fluorescent tubes or similar means, including white light LEDs, allows permanent and continuous production of the cyanobacteria, which is strictly necessary for industrial level production of neosaxitoxin, the main compound to be purified from these cyanobacteria. However, depending on the cultured cyanobacteria and the culture conditions, not only neosaxitoxin can be purified later on, but also saxitoxin and other saxitoxin analogs, such as gonyaulatoxins and others. All these compounds belong to the group of the paralyzing phycotoxins.

The process of continuous industrial production with high cyanobacteria yields has not been carried out previously. Until now, no development of an artificial procedure for cyanobacteria having a characteristic and unique phycotoxin profile has been described; for example, a unique composition containing only neosaxitoxin and saxitoxin in a predefined 5 to 1 ratio respectively, or only gonyaulatoxins without any other paralyzing phycotoxins.

Another advantageous characteristic is the very high production of phycotoxins obtained when using the selected culture conditions and procedures that are described in the present invention, which allows cyanobacteria to be defined as the best source of these phycotoxins, especially neosaxitoxin in the case of *Aphanizomenon(Aph) gracile* (Lemm) Lemm, which is the major component and the most interesting one to be massively produced.

This species of cyanobacteria, *Aphanizomenon(Aph) gracile* (Lemm) Lemm, a producer of saxitoxin and mainly neosaxitoxin, was collected from Crato Lake, a water reservoir in Portugal, isolated and codified as **LMECYA 41** and was identified as *Aphanizomenon (Aph) gracile* (Lemm) Lemm, firstly taking into account its morphology and later based on its 16S rRNA gene sequence (Paulo Pereira, et al., 2001. 5th International conference on toxic cyanobacteria, July 15-20 2001, Queensland, Australia).

Initially, this strain was collected in a crude water sample collected from the reservoir, which had a high predominance of *Aphanizomenon (Aph) gracile* (Lemm) Lemm filaments, the second predominating species, with 32 to 40% of the total mass of species found in the original sample. Later, in the laboratory, conventional routine techniques were used to repetitively and successively wash and isolate trichomas individually in cloning droplets taken with clamps (small sterile isolation rings), all in sterile culture media and being observed with the help of an inverted microscope, to obtain a single trachoma that was transferred to a sterile 10-milliliter flask containing 5 milliliters of medium. The culture of this single cyanobacterium (unialgal culture) was grown at 25°C for 2 to 4 weeks, until reaching a suitable development state for subsequent production. From this original isolate, massive expansion and production of this unialgal cyanobacterium was started by doing continuous replications as required, using always the count of filaments maintained as a guide, and always in the exponential growing phase. During this growth and development stage of the unialgal culture, the strain was kept under light-darkness cycles of 16:8 hours. The filaments were counted using lugol stain in Sedgewick Rafter and Palmer-Maloney counting cells, thereby controlling the development of the culture.

This method to isolate a cyanobacterium to get a pure culture can be applied to any of the cyanobacteria able to produce paralyzing phycotoxins mentioned above.

Once the pure culture is obtained, it is used as an inoculum for massive production of cyanobacteria according to the present invention.

The advantages of the present invention to massively produce cyanobacteria that are able to produce paralyzing phycotoxins under controlled conditions are:
- Continuous and permanent production under controlled conditions of cyanobacteria that are able to produce paralyzing phycotoxins.
- Low production costs because the culture medium is very elemental, since it does not include highly-valued nutrients and only requires light control, which means that handling of cyanobacteria is relatively easy.
- Providing a continuous source of paralyzing phycotoxins (saxitoxin, neosaxitoxin and gonyaulatoxins) independent from environmental conditions and changes.
- Very favorable cost-production-yield relationship that has not been achieved previously and has not been described in any industrial process until now.

The document by Pomati et al, Pomati et al: "Evidence for differences in the metabolism of saxitoxin and C1+2 toxins in the freshwater cyanobacterium Cylindrospermopsis raciborskii T3", BIOCHIMICA ET BIOPHYSICA ACTA - GENERAL SUBJECTS, ELSEVIER SCIENCE PUBLISHERS, NL, vol. 1674, no. 1, 6 September 2004 (2004-09-06), pages 60-67, discloses the growth of cyanobacteria *Cylindrospermopsis raciborskii* in MLA medium supplemented with 10 mM of arginine and discloses that said arginine levels increase the production of saxitoxin in *Cylindrospermopsis raciborskii* strain T3.

The document by Pomati et al, Pomati et al: "The purine degradation pathway: Possible role in paralytic shellfish toxin metabolism in the cyanobacterium Planktothrix sp", ENVIRONMENT INTERNATIONAL, PERGAMON PRESS, US, vol. 27, no. 6, 1 December 2001 (2001-12-01), pages 463-470, discloses that allantoic acid stimulates the production of saxitoxin in *Planktothrix* species.

The document by Soto et al, Soto et al: "The effects of chloramphenicol, arginine and temperature on PST-production by Cylindrospermopsis raciborskii strain D9", PROCEEDINGS OF THE 12TH INTERNATIONAL CONFERENCE ON HARMFUL ALGAE. COPENHAGEN 2008, INTERNATIONAL SOCIETY FOR THE STUDY OF HARMFUL ALGAE AND INTERGOVERNMENTAL OCEANOGRAPHIC COMMISSION OF UNESCO, 1 January 2008 (2008-01-01), pages 330-333, discloses that the D9 strain of *Cylindrospermopsis raciborskii* has a 48% decrease in saxitoxin production upon arginine stimulation (in MLA medium) while the T3 strain of the same species has a 476% increase.

The documents by Shimizu et al, Shimizu et al: "BIOSYNTHESIS OF SAXITOXIN ANALOGS THE UNEXPECTED PATHWAY", JOURNAL OF THE AMERICAN CHEMICAL SOCIETY, vol. 106, no. 21, 1984, pages 6433-6434, and by Quintero et al, Quintero et al: "Arginine catabolism in the cyanobacterium Synechocystis sp. Strain PCC 6803 involves the urea cycle and arginase pathway.", JOURNAL OF BACTERIOLOGY, vol. 182, no. 4, 1 February 2000 (2000-02-01), pages 1008-1015, discloses that in order to build a saxitoxin molecule, molecules of arginine, acetate and methionine are needed.

### OBJECT OF THE INVENTION

The general object of the present invention is the massive continuous industrial production of a cyanobacterium that produces mainly paralyzing phycotoxins, such as saxitoxin, neosaxitoxin and/or gonyaulatoxins, under controlled conditions and at low cost.

A specific object of the present invention is the development of a procedure for optimized production of a cyanobacterial monoculture at low cost and commercially competitive.

Another specific objective of the invention is the achievement of continuous industrial procedure using a cyanobacteria strain in perpetuity as a source of high added value compounds that are not commercially available and have applications as clinical therapies for several pathologies.

Another specific object of the invention is the achievement of a continuous culture of this cyanobacterial strain directed to produce mainly neosaxitoxin, with a small proportion of saxitoxin (one fifth saxitoxin), or mainly gonyaulatoxins 2/3 as major and main components.

Another specific objective of the invention is to achieve a high production of neosaxitoxin and/or gonyaulatoxins per cyanobacterial cell.

### BRIEF DESCRIPTION OF THE DRAWINGS

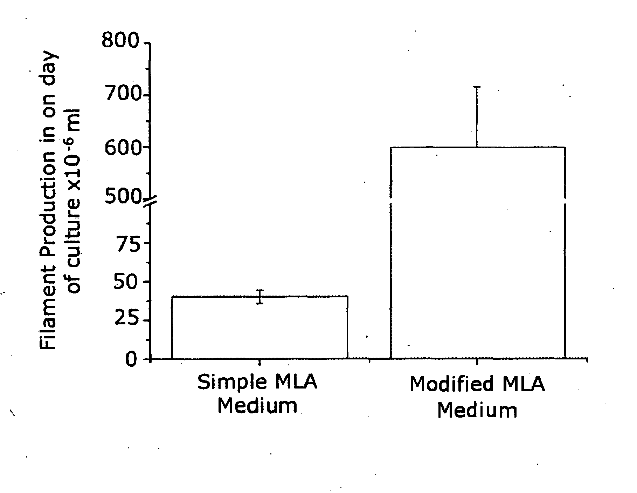

FIGURA 1: Production of filaments in one culture day using the culture medium of the present invention (MLA Medium modified with methionine, arginine and allantoic acid) in continuous light conditions and the unmodified medium (simple MLA Medium) with light:darkness cycling.

### BRIEF DESCRIPTION OF THE INVENTION

In the present application, a method to massively obtain and produce cyanobacteria in a continuous culture is presented. The examples of cyanobacteria that can be cultured continuously are:
- *Cylindrospermopsis raciborskii, Aphanizomenon flos-aquae;*
- *Aphanizomenon(Aph) issatschenkoi (Usacev) Proskina-Lavrenco;*
- *Anabaena circinalis, Lyngbya wollei* and *Aphanizomenon gracile (Lemm) Lemm.*

The knowledge about cyanobacteria that produce paralyzing phycotoxins is associated to a public health issue, wherein shellfish contamination with these phycotoxins, related to blooms of phycotoxin-producing dinoflagellates, has a serious impact on public health and the shellfish canning industry in regions where red tides are present, *i.e.* where phycotoxin-producing dinoflagellates blooms appear (Lagos 1998).

These cyanobacteria species, when described for the first time, were characterized only according to anatomy and classical taxonomical characteristics. These species were not initially studied or characterized according to the compounds they produce, and when the study of the produced phycotoxins was carried out, the objective was to understand and solve the toxicity levels and public health problems. However, until the presentation of this application, a massive production of paralyzing phycotoxins (all saxitoxin analogs) derived from cyanobacteria or the culture of cyanobacteria as a continuous and perpetual source of paralyzing phycotoxins have never been proposed.

In the present application, a method to massively obtain and produce cyanobacteria in a continuous culture is presented, taking as an example the culture of *(Aph) gracile* (Lemm) Lemm. However, the continuous culture method is applicable to the cyanobacteria genera mentioned previously> *Cylindrospermopsis raciborskii, Aphanizomenon flos-aquae, Aphanizomenon(Aph) issatschenkoi (Usacev) Proskina-Lavrenco, Anabaena circinalis, Lyngbya wollei,* and Aphanizomenon *gracile (Lemm) Lemm.*

The availability of these isolated cyanobacteria has allowed the development of continuous studies and the invention of the present massive production process

To culture and develop a cyanobacterium, serial open or closed reactors are required, and direct illumination is needed to coat all the reactors.

The culture only requires fresh water, mineral salts and micronutrients in low amounts, all of them with low costs and readily prepared.

Another preferred requirement is a permanent light cycle with no darkness period, which also constitutes an innovation of this massive culture procedure, since cyanobacteria are photosynthetic organisms that require light and reach maximal development always in the exponential growth phase. In relation to this, it is worth highlighting that light and darkness cycles with different hourly regimes can also be used during the development of this continuous culture, even natural light cycles. Another innovation of the process described in this application is the permanent maintenance of the culture in the exponential growth phase and in a permanent state of production of phycotoxins, which is achieved by carrying out selective quantified harvests, depending on the quantitative development of the culture in each reactor.

The culture of cyanobacteria requires sterile media to keep the cultures in the best conditions; however these cultures do not require extreme sterility conditions as in the case of the culture of eukaryotic cells such as mammal cells.

These cyanobacteria are very resistant to contamination and since their development requirements are minimal and therefore the culture media used in the present invention are too "poor" for the development of other microorganisms, these are natural selection media where only these photosynthetic organisms can grow.

The optimal range of temperature for the growth of these cyanobacteria is comprised between the usual ranges of variation of the environmental temperature variations in a Mediterranean climate such as Central Chile (from Arica to Temuco).

When cyanobacteria have developed, a volume suitable to be used, for example, for purification of a metabolite produced by the cyanobacteria, is removed and the volume removed from the cell culture is restituted by adding an equivalent volume of sterilized medium. According to the filament counting in time, once a determined development has been reached, another suitable volume of cyanobacteria can be collected, always keeping the same final volume of the culture medium by replacing the harvested volume of cells by an equivalent refill volume.

In this way, a system of cultured cyanobacteria is obtained as a continuous source for production of cyanobacteria.

### DETAILED DESCRIPTION OF THE INVENTION

To start a culture of a cyanobacterium able to produce paralyzing phycotoxins at industrial levels, an isolated and characterized cyanobacterium is required, which can be selected from *Cylindrospermopsis sp., Aphanizomenon sp., Anabaena sp.,* and *Lyngbya sp.,* and the like.

The culture of cyanobacteria, considering a particularly selected species, is carried out in suitable aerated stirred reactors with permanent illumination. Light can be artificial, e.g. light emitted by fluorescent tubes.

The culture medium considered for the reproduction of cyanobacteria comprises distilled water, mineral salts and micronutrients, all sterile.

The culture medium is prepared based on the MLA X 40 medium (MLA medium, CSIRO Marine Research, CSIRO Microalgae Research Center, Hobart, Australia) for cyanobacterial culture (microalgae@marine.csiro.au), which comprises, among others, the following components: MgSO₄ × 7H₂O, NaNO₃, K₂HPO₄, H₃BO₃, H₂SeO₄, biotin, vitamin B12 and thiamine-HCl, EDTA-Na, ferric chloride hexahydrate, sodium carbamate and manganese chloride hydrate, NaHCO₃, CaCl₂ × 2H₂O, CuSO₄ × 5H₂O, ZnSO₄ × 7H₂O, CoCl₂ × 6H₂0, NaMoO₄ × 2H₂0. Furthermore, the culture medium of the present invention incorporates arginine, methionine and allantoic acid.
In the following table, concentration ranges useful for the method of the present invention are presented for the components mentioned above.

| Compound | Minimal concentration (g/l) | Maximal concentration (g/l) |
|---|---|---|
| MqSO₄ × 7H₂O | 3.71E-02 | 6.18E-02 |
| NaNO₃ | 1.28E-01 | 2.13E-01 |
| K₂HPO₄ | 2.61E-02 | 4.35E-02 |
| H₃BO₃ | 1.85E-03 | 3.09E-03 |
| H₂SeO₄ | 9.68E-04 | 1.61E-03 |
| Biotin | 3.75E-08 | 6.25E-08 |
| Vitamin B12 | 3.75E-08 | 6.25E-08 |
| Thiamine HCl | 7.50E-05 | 1.25E-04 |
| CuSO₄ × 5H₂O | 7.50E-06 | 1.25E-05 |
| ZnSO₄ × 7H₂O | 1.65E-05 | 2.75E-05 |
| CoCl₂ × 6H₂0 | 7.50E-06 | 1.25E-05 |
| NaMoO₄ × 2H₂0 | 4.50E-06 | 7.50E-06 |
| Na₂EDTA | 3.27E-03 | 5.45E-03 |
| FeCl₃ × 6H₂O | 1.19E-03 | 1.98E-03 |
| NaHCO₃ | 4.50E-04 | 7.50E-04 |
| MnCl₂ × H₂O | 2.70E-04 | 4.50E-04 |

Additionally, the culture medium comprises from 2 to 3.5 mM of arginine, from 1 to 2.2 mM of methionine and from 0.7 to 1.3 mM of allantoic acid in the final composition.

The preparation of the culture medium is carried out using stock solutions that are separately prepared and sterilized by filtration or autoclaving.

The MLA culture medium from CSIRO for cyanobacterial growth is known, however the complete culture medium described in the present invention considers mineral salts, micronutrients and vitamins that are not found in said medium or in any other publication regarding cyanobacterial culture.

Once the medium is prepared, cyanobacteria are inoculated ranging from 20 to 40 million filaments per each 3 liters of the culture medium of the invention.

The culture conditions consider constant illumination provided by fluorescent tubes at a controlled temperature from 15 to 35°C, preferably in a range from 20 to 35°C, with an optimum of 22 ± 2°C.

In particular, depending on the division cycle of the strain used in the culture, it is possible to remove a volume from 20 to 40% of the total volume of the reactor to process cyanobacteria and replace this volume with an equivalent volume of fresh culture medium. For example, if the division cycle is 0.33 per day, it is possible to remove one third of the culture volume and replace it with an equivalent volume of fresh culture medium every 1 to 2 days.

To determine the moment to harvest cyanobacteria, growth is monitored through filament counting and care is taken to restitute an equivalent volume of culture medium each time a removal of cyanobacteria is carried out.

There is a previous publication wherein the culture of the strain *Aphanizomenon (Aph) gracile (Lemm)* Lemm **LMECYA40** is described, but the culture medium described in this publication is totally different from the modified MLA medium described in this invention. This is the first time the use of MLA medium is described as a base for the culture of cyanobacteria. To adapt the culture of cyanobacteria of the present invention, the MLA medium has been modified adding the components arginine, methionine and allantoic acid, all compounds that have surprisingly demonstrated to be highly potent stimulants of the production of the phycotoxins saxitoxin, neosaxitoxin and gonyaulatoxins.

In the first step, the method of the invention comprises generating an inoculum with a number of filaments in the range from 20 to 40 million, cultured in small 250-ml flasks in a culture chamber with a light:darkness cycling of 16:8 hours and temperature controlled at 22 ±2°C.

The inoculum obtained is transferred into reactors, which are in kept in sterile and isolated conditions.

The culture temperature of the method of the present invention is controlled to be between 20 - 25°C. For example, if *Aphanizomenon (Aph) gracile* (Lemm) Lemm is going to be produced, it has to be taken into account that this strain is well cultured in the range from 15 to 30°C, optimally 22 ± 2 °C.

*Aphanizomenon (Aph) gracile* (Lemm) Lemm cells have a previously known division cycle of 0.33 per day. This means that in three days the culture doubles the cell mass. This allows the collection of one liter of culture from each 3-liter reactor every 1 to 2 days maximum.

The volume removed from the cell culture is restituted adding one liter of sterilized medium. The next day or the other, according to the filament counting, another liter of cyanobacterial culture can be collected, always keeping a total volume of 3 liters of culture medium.

In this way, a system of cultured cyanobacteria is obtained as a continuous source for perpetual production of cyanobacteria. The collection of culture medium with cyanobacteria can be conveniently carried out every two days, thereby obtaining a larger number of filaments. Under maximal production conditions, it is not convenient to space harvesting for more than 4 days, since the culture deteriorates and phycotoxin production decrease when filament concentration in the culture increases.

The cell pellet corresponds to filament-forming cyanobacteria. These filaments have from 20 to 100 cells and even more cells linked in a filament. For that reason, they are called filamentous cyanobacteria. When the cyanobacteria are "healthy" and grow well, they form filaments, because they undergo cell division therein. This filamentous characteristic is used as growth control, because it is a positive cyanobacterial development control. The ability to form filaments allows a better control of medium contamination, since when the medium is contaminated and an infection arises, cyanobacteria do not form filaments.

The cell pellet comprises filaments that are quantified in the inverted microscope, since individual cells are very small, difficult to count and are not present in a healthy culture.

Furthermore, cell-free medium is collected as the supernatant of a centrifugation step used to obtain a pellet. This centrifugation is conveniently performed at 10,000 g for 20 minutes. Phycotoxins are also present as soluble species in this supernatant.

This supernatant is the second phycotoxin source. In good culture conditions, with healthy filaments, no contamination and in permanent exponential growth phase, it tends to be less than 5% of the total content obtained in the pellet fraction.

The productivity described in the present invention is between 60-125 times higher than productivities described by Paulo Pereira et al., 2001, (5th International Conference on Toxic Cyanobacteria, July 15-20 2001, Queensland, Australia).

The strain was cultured in conventional conditions (without the reactors developed and used in this invention), with other nutrients and micronutrients, keeping light:darkness cycles of 16:8 hours, and at other temperatures. Certainly, in the present invention a massive industrial production is described that includes not only a new continuous culture method, but also a procedure using a novel infrastructure, which as a whole delivers an innovative proposal with a surprising effect due to the high yields that have never been described or demonstrated before.

Although the previous description contains many specifications, these specifications must not be taken as limiting the scope of the invention, but only as illustrations of some of the modalities of the invention that are actually preferred.

The developed pilot industrial process is a continuous culture comprising 200 reactors that allow the daily collection of 200 liters of cyanobacteria able to produce paralyzing phycotoxins (neosaxitoxin, saxitoxin or gonyaulatoxins).

### EXAMPLE 1: Preparation of culture media

To prepare the culture medium, more concentrated stock solutions of micronutrients, mineral salts and vitamins are prepared. All solutions are prepared with distilled water.
The Vitamin Stock (solution A) is prepared according to the directions in the following table:

| | Concentration (mg/ml) | Volume (ml) | Final concentration (mg/ml) |
|---|---|---|---|
| Biotin | 0.1 | 0.05 | 0.00005 |
| Vitamin B12 | 0.1 | 0.05 | 0.00005 |
| Thiamine HCl | | | 0.1 |

In other words, 1 mg of biotin was dissolved in 10 ml of distilled water. Similarly, 1 mg of vitamin B12 was dissolved in 10 ml of distilled water. 0.05 ml from each of these two solutions were taken and mixed with 10 mg of thiamine HCl in a final volume of 100 ml completed with distilled water.

The Micronutrient Stock (solution B) was prepared from primary solutions of CuSO₄ · 5H₂O (1 g/l), ZnSO₄ · 7H₂O (2.2 g/l), CoCl₂ · 6H₂O (1 g/l), NaMoO₄ · 2H₂0 (0.6 g/l).
To 800 ml of a solution as indicated in the table below, 10 ml of each primary solution were added.

| | Concentration (g/l) | Amount (g) in 800 ml |
|---|---|---|
| Na₂EDTA | 5.45 | 4.36 |
| FeCl₃ · 6H₂O | 1.975 | 1.58 |
| NaHCO₃ | 0.75 | 0.6 |
| MnCl₂ · H₂O | 0.45 | 0.36 |

Finally, the volume was completed up to 1 liter with distilled water to get 1 liter of Micronutrient Stock Solution (solution B).

To prepare 250 ml of a concentrated 40x stock of MLA medium, the volumes indicated for each component in the following table were added to 130 ml of distilled water:

| Component | Solution (g/l) | Volume (ml) |
|---|---|---|
| MgSO₄ · 7H₂O | 49.4 | 10 |
| NaNO₃ | 85 | 20 |
| K₂HPO₄ | 6.96 | 50 |
| H₃BO₃ | 2.47 | 10 |
| H₂SeO₄ | 1.29 | 10 |
| Vitamin Stock (solution A) | | 10 |
| Micronutrient Stock (solution B) | | 10 |

Finally, to prepare one liter of MLA culture medium to be sterilized by filtration through 0.22 micrometer membranes, the following volumes are combined:

| | (g/l) | Volume (ml) |
|---|---|---|
| Distilled water | | 964 |
| 40× MLA Stock | | 25 |
| NaHCO₃ | 16.9 | 10 |
| CaCl₂ · 2H₂O | 29.4 | 1 |

In case of using autoclave sterilization at 121°C for 15 minutes, the amounts indicated below are used, and pH is adjusted to 7.5 - 8.0 with HCl or other suitable acid.

| | (g/l) | Volume (ml) |
|---|---|---|
| Distilled water | | 973 |
| 40× MLA Stock | | 25 |
| NaHCO₃ | 16.9 | 1 |
| CaCl₂ · 2H₂O | 29.4 | 1 |

The concentrations of the final components are those indicated in the following table:

| Compound | Concentration in the ready-to-use medium (g/l) |
|---|---|
| MgSO₄ · 7H₂O | 4.94E-02 |
| NaNO₃ | 1.70E-01 |
| K₂HPO₄ | 3.48E-02 |
| H₃BO₃ | 2.47E-03 |
| H₂SeO₄ | 1.29E-03 |
| Biotin | 5.00E-08 |
| Vitamin B12 | 5.00E-08 |
| Thiamine HCl | 1.00E-04 |
| CuSO₄ · 5H₂O | 1.00E-05 |
| ZnSO₄ · 7H₂O | 2.20E-05 |
| CoCl₂ · 6H₂0 | 1.00E-05 |
| NaMoO₄ · 2H₂0 | 6.00E-06 |
| Na₂EDTA | 4.36E-03 |
| FeCl₃ · 6H₂O | 1.58E-03 |
| NaHCO₃ | 6.00E-04 |
| MnCl₂ · H₂O | 3.60E-04 |

NaHCO₂ and CaCl₂ · 2H₂O must be added as needed, since their concentration depends on the type of sterilization (filtration or autoclaving) used for the medium.

All the solutions can be stored refrigerated for no more than 1 month.

Finally, to prepare the culture medium of the present invention, arginine, methionine and allantoic acid were added at final concentrations of 2.8 mM arginine, 1.7 mM methionine and 1 mM allantoic acid.

This final mixture of added components is determinant to obtain the results of this invention and said components are not part of the MLA Medium (CSIRO Marine Research) described above.

### EXAMPLE 2: Culture of Aphanizomenon (Aph) gracile (Lemm) Lemm

Each reactor containing 3 liters of the culture medium described in Example 1 was inoculated with 35 million filaments of *Aphanizomenon (Aph) gracile* (Lemm) Lemm. The reactors were kept at a controlled temperature of 22°C ± 2°C, with permanent illumination from fluorescent tubes. Once the culture reached the exponential phase, one-third of the total reactor volume was collected (1 liter) and replaced with 1 liter of fresh medium. The former culture had a doubling time of 0.33 times per day.
Subsequently, the culture medium with cyanobacteria was centrifuged at 10,000 g for 20 minutes.

The yields obtained in the present invention are always expressed as a function of the number of filaments, since the presence of filaments (association of many individual cells) is an indicator of the quality of the culture and of the permanent reproduction and development of the cells. A filament-rich culture is a "healthy" culture and is in permanent development. The cell pellet is a pellet of filaments and the purification of phycotoxins starts therefrom.

**TABLE 1: Number of Aphanizomenon (Aph) gracile (Lemm) filaments per milliliter of culture as a function of time**

| **Batch** | **Culture day** | **Filaments of cyanobacteria in the pellet per ml** | **Collected volume (ml)** |
|---|---|---|---|
| 1 | Day 1 | 590,000 | 1000 |
| 2 | Day 2 | 598,000 | 1000 |
| 3 | Day 3 | 630,000 | 1000 |
| 4 | Day 4 | 596,000 | 1000 |
| 5 | Day 5 | 626,000 | 1000 |
| 6 | Day 6 | 788,000 | 1000 |
| 7 | Day 7 | 534,000 | 1000 |
| 8 | Day 8 | 756,000 | 1000 |
| 9 | Day 9 | 754,000 | 1000 |
| 10 | Day 10 | 614,000 | 1000 |
| 11 | Day 11 | 622,000 | 1000 |
| 12 | Day 12 | 608,000 | 1000 |
| 13 | Day 13 | 770,800 | 1000 |

The cell pellet of each batch is the volume obtained from one liter of culture harvested each day from the reactor. This can also be carried out every other day, to achieve a larger number of filaments. Under these maximal production conditions, it is preferable to carry out the harvest no more than 4 days apart, to avoid culture deterioration and phycotoxin production decrease.

### EXAMPLE 3: Comparison of the culture with simple (unmodified) MLA medium and the modified MLA medium of the present invention.

Figure 1 shows the difference between a culture of *Aphanizomenon gracile* in the simple (unmodified) MLA medium and a culture of *Aphanizomenon gracile* with the modified MLA culture medium of the present invention. It is easily appreciable that the modified medium of the present invention, including a final concentration of 2.8 mM arginine, 1.7 mM methionine and 1 mM allantoic acid, is surprisingly superior to the culture using the simple (unmodified) MLA medium.

Table 2 below shows the concentration of the different phycotoxins in the supernatant and the yield of cyanobacterial filaments thereof. The table also indicates the concentration of filaments per ml of culture medium according to the method of the present invention.

**TABLE 2: Production of neosaxitoxin and saxitoxin as a function of the number of filaments and the wet weight of the Aphanizomenon gracile pellet grown in modified MLA medium (with arginine, methionine and allantoic acid).**

| Concentration in the supernatant | | | | Concentration of phycotoxin per filament | | Cyano. fil./ml* |
|---|---|---|---|---|---|---|
| neoSTX mM | STX mM | neoSTX *µ*g/ml | STX *µ*g/ml | neoSTX pg/fil. | STX pg/fil | |
| 8.98 | 3.5 | 2.83 | 1.05 | 2.42 | 0.9 | 1,170,000,00 |
| 13.66 | 4.91 | 4.3 | 1.47 | 3.44 | 1.18 | 1,250,000,00 |
| 3.42 | 1.03 | 1.08 | 0.31 | 1.71 | 0.49 | 630,000,00 |
| 17.82 | 4.78 | 5.61 | 1.43 | 9.42 | 2.41 | 596,000,00 |
| 17.81 | 4.02 | 5.61 | 1.21 | 13.17 | 2.83 | 426,000,00 |
| 7.66 | 1.47 | 2.41 | 0.44 | 4.94 | 0.91 | 488,000,00 |
| 10.87 | 2.19 | 3.42 | 0.66 | 10.25 | 1.97 | 334,000,00 |
| 10.52 | 2.07 | 3.31 | 0.62 | 4.38 | 0.82 | 756,000,00 |
| 10.77 | 1.96 | 3.39 | 0.59 | 7.47 | 1.3 | 454,000,00 |
| 17.24 | 2.99 | 5.43 | 0.9 | 13.12 | 2.16 | 414,000,00 |
| 5.31 | 0.41 | 1.67 | 0.12 | 5.19 | 0.38 | 322,000,00 |
| 12.26 | 2.17 | 3.86 | 0.65 | 9.47 | 1.59 | 408,000,00 |
| 9.84 | 1.94 | 3.1 | 0.58 | 17.42 | 3.27 | 178,000,00 |

| | | | | | | |
|---|---|---|---|---|---|---|
| STX: saxitoxin neoSTX: neosaxitoxin Cyano. fil.: cyanobacterial filaments * Cyanobacterial filaments are associations of 20 to 100 cyanobacterial cells. These cyanobacteria form filaments in solution and those filaments are counted using the magnification of an inverted microscope. | | | | | | |

Table 3 shows different phycotoxin production batches. It is clearly observed that the mean yield of phycotoxin per filament is much higher when using the modified MLA medium with the growth conditions of the present invention, in comparison with the culture in simple (unmodified) MLA medium representing the previous state of the art.

**TABLE 3: Production of neosaxitoxin and saxitoxin as a function of the number of filaments in the modified MLA medium (of the invention) and the simple MLA medium.**

| | Production of phycotoxins in modified MLA medium and permanent illumination | | Production of phycotoxins in simple MLA medium with light:darkness cycling | |
|---|---|---|---|---|
| | neoSTX pg/fil. | STX pg/fil | neoSTX pg/fil. | STX pg/fil |
| | 2.42 | 0.9 | 0.09 | 0.03 |
| | 3.44 | 1.18 | 0.13 | 0.04 |
| | 1.71 | 0.49 | 0.06 | 0.02 |
| | 9.42 | 2.41 | 0.35 | 0.09 |
| | 13.17 | 2.83 | 0.49 | 0.1 |
| | 4.94 | 0.91 | 0.18 | 0.03 |
| | 10.25 | 1.97 | 0.38 | 0.07 |
| | 4.38 | 0.82 | 0.16 | 0.03 |
| | 7.47 | 1.3 | 0.28 | 0.05 |
| | 13.12 | 2.16 | 0.48 | 0.08 |
| | 5.19 | 0.38 | 0.19 | 0.01 |
| | 9.47 | 1.59 | 0.35 | 0.06 |
| | 17.42 | 3.27 | 0.65 | 0.12 |
| **Average** | **7.88** | **1.55** | **0.29** | **0.06** |

The yields obtained in these experiments show a surprising and unexpected production of pure phycotoxins (neosaxitoxin and saxitoxin) per cyanobacterial filament cultured in the modified MLA medium (Table 2), when compared to the production of the cyanobacterial filaments cultured with usual conditions (simple MLA medium) and described in the state of the art in small culture flasks, with no continuous aeration and with light (day): darkness (night) cycles (Table 3).

In the example described herein, cyanobacteria are always in the logarithmic growth phase, with permanent illumination 24 hours a day and with permanent collection of cyanobacteria, inducing permanent growth by adding new nutrients in a volume that is equivalent to the volume collected in each harvest.

As demonstrated' in Table 3, the method of the invention achieves better yields that are approximately 25 times greater than those of known methods.

## Claims

1. Method to obtain and to produce massively an isolated species of cyanobacteria that produces paralyzing phycotoxins selected from the group consisting of Cylindrospermopsis raciborskii, Aphanizomenon flos-aquae, Aphanizomenon (Aph) issatschenkoi (Usacev) Proskina-Lavrenco, Anabaena circinalis, Lyngbya wollei and Aphanizomenon gracile (Lemm) Lemm, **characterized in that** said method comprises the steps of:
a. inoculating from 20 to 40 million cyanobacterial filaments every 3 liters of MLA culture medium comprising MgSO₄ · 7H₂O, NaNO₃, K₂HPO₄, H₃BO₃, H₂SeO₄, biotin, vitamin B12, thiamine HCl, CuSO₄ · 5H₂O, ZnSO₄ · 7H₂O , CoCl₂ · 6H₂0, NaMoO4 · 2H₂0 , Na₂EDTA, FeCl₃ · 6H₂O, NaHCO₃, MnCl₂ · H₂O, supplemented with from 2 to 3.5 mM arginine, from 1 to 2.2 mM methionine and 0.7 to 1.3 mM allantoic acid;
b. culturing the cyanobacteria in the culture medium at a temperature from 15 to 35°C under permanent natural or artificial illumination;
c. collecting a volume of culture medium ranging from 20 to 40% of the total volume every 1 to 3 days and restituting the removed volume with fresh culture medium;
d. keeping the culture in continuous mode;
e. centrifugating the medium with cyanobacteria collected in c) at a relative centrifugal force ranging from 5,000 to 15,000 × g for a period from 5 to 30 minutes and obtaining said cyanobacteria from the pellet resulting from the centrifugation.

2. The method according to claim 1, in which the culture temperature is in the range from 15 to 25°C.

3. The method according to Claim 2, in which said temperature is of 22±2°C.

4. The method according to claim 1, in which the culture medium has a final arginine concentration of 2.8 mM.

5. The method according to claim 1, in which the culture medium has a final methionine concentration of 1.7 mM.

6. The method according to claim 1, in which the culture medium has a final allantoic acid concentration of 1.0 mM.

## Patentansprüche

1. Verfahren, um eine isolierte Art von Cyanobakterien zu erhalten und massiv zu produzieren, die eine lähmende Phycotoxine produziert, ausgewählt aus der Gruppe bestehend aus Cylindrospermopsis raciborskii, Aphanizomenon flos-aquae, Aphanizomenon (Aph) issatschenkoi (Usacev) Proskina-Lavrenco, Anabaena circinalis, Lyngbya wollei und Aphanizomenon gracile (Lemm) Lemm, **dadurch gekennzeichnet, dass** das genannte Verfahren folgende Schritte umfasst:
a. Impfen from 20 - 40 Millionen Cyanobakterien Fäden alle 3 Liter MLA Kulturmedium mit MgSO₄ · 7H₂O, NaNO₃, K₂HPO₄, H₃BO₃, H₂SeO₄, Biotin, Vitamin B12, Thiamin-HCl, CuSO₄ · 5H₂O, ZnSO₄ · 7H₂O, CoCl₂ ·6H₂O, NaMoO₄ · 2H₂O, Na₂EDTA, FeCl₃ · 6H₂O, NaHCO₃, MnCl₂ · H₂O, ergänzt mit 2 bis 3,5 mM Arginin, mit 1 bis 2,2 mM Methionin und 0,7 bis 1,3 mM Allantoinsäure;
b. Kultivieren der Cyanobakterien in dem Kulturmedium bei einer Temperatur von 15 bis 35 ° C unter natürliche oder künstliche Dauerbeleuchtung;
c. Sammeln des Kulturmediums im Bereich von 20 bis 40% des Gesamtvolumens alle 1 bis 3 Tageund Ersetzen des entfernten Volumen mit frischem Kulturmedium;
d. Halten der Kultur im kontinuierlichen Modus;
e. Zentrifugation des Mediums mit der in c) gesammelten Cyanobakterien bei einer relativen Zentrifugalkraft von 5000 bis 15000 x g für einen Zeitraum von 5 bis 30 Minuten und Erhalten der Cyanobakterien aus dem Pellet der resultierendne Zentrifugation.

2. Verfahren nach Anspruch **1**, wobei die Kulturgrenztemperatur im Bereich von 15 bis 25° C liegt.

3. Verfahren nach Anspruch 2, wobei die Temperatur bei 22±2 ° C liegt.

4. Verfahren nach Anspruch 1, wobei das Kulturmedium eine endgültige Arginin-Konzentration von 2,8 mM hat.

5. Verfahren nach Anspruch 1, wobei das Kulturmedium eine endgültige Methionin-Konzentration von 1,7 mM hat.

6. Verfahren nach Anspruch 1, wobei das Kulturmedium eine endgültige Allantoinsäure Konzentration von 1,0 mM hat.

## Revendications

1. Méthode pour obtenir et à produire massivement une espèce isolées de cyanobactéries qui produisent des phycotoxines paralysantes choisies dans le groupe constitué de Cylindrospermopsis raciborskii, Aphanizomenon flos-aquae, Aphanizomenon (Aph) issatschenkoi (Usacev) Proskina-Lavrenco, circinalis Anabaena, Lyngbya wollei et Aphanizomenon gracile (Lemm) Lemm, **caractérisé en ce que** ledit procédé comprend les étapes consistant à :
a. inoculer entre 20 à 40 millions de filaments de cyanobactéries tous les 3 litres de milieu de culture MLA comprenant Mg SO₄ · 7H ₂O, NaNO₃, K₂HPO₄, H₃BO₃, H₂SeO₄, biotine, vitamine B12, thiamine HCI, CuSO₄,5H₂O, ZnSO₄, 7H₂O₄, CoCl₂ 6H₂0, NaMoO₄, 2H₂0, Na₂EDTA, FeCl₃ 6H₂O, NaHCO₃, MnCl₂ · H₂0, complété par mM arginine de 2 à 3,5, d'arginine 1 à 2,2 mM de méthionine et 0,7 à 1,3 mM d'acide allantoïdien;
b. la mise en culture des cyanobactéries dans le milieu de culture à une température de 15 à 35° C sous éclairage naturel ou artificiel permanent ;
c. la collecte d'un volume de milieu de culture compris entre 20 et 40% du volume total tous les 1 à 3 jours et restituer le volume enlevé par du milieu de culture frais;
d. maintenir la culture en mode continu ;
e. centrifuger le milieu avec les cyanobactéries collectées en c) à une force centrifuge relative allant de 5000 à 15 000 x g pendant une durée de 5 à 30 minutes, et obtenir ladite cyanobactérie à partir du fragment issu de la centrifugation.

2. Procédé selon la revendication **1**, dans laquelle la température de culture est de l'ordre de 15 à 25° C.

3. Procédé selon la revendication 2, dans laquelle ladite température est de 22+/-12° C.

4. Procédé selon la revendication 1, dans laquelle le milieu de culture présente une concentration en arginine finale de 2,8 mM.

5. Procédé selon la revendication 1, dans lequel le milieu de culture présente une concentration en méthionine finale de 1,7 mM.

6. Procédé selon la revendication 1, dans lequel le milieu de culture présente une concentration en acide allantoïdien finale de 1,0 mM.
